# EUROPEAN PATENT SPECIFICATION

(11) **EP 2 253 329 B1**
(45) Date of publication and mention of the grant of the patent: **21.09.2016**
(21) Application number: 09380086.0
(22) Date of filing: 27.04.2009
(51) Int. Cl.: A61K 47/40, A61K 47/48, A61K 31/192

(54) **Ibuprofen lysinate oral suspension**
Orale Ibuprofenlysinat-Suspension
Suspension orale d'Ibuprofène lysinate.

(43) Date of publication of application: 24.11.2010
(73) Proprietor: Laboratorio de Aplicaciones Farmacodinamicas, S.A., 08025 Barcelona (ES)
(72) Inventor: Tarré Pérez, Maite, 08005 Barcelona (ES)
(74) Representative: Carvajal y Urquijo, Isabel

(56) References cited:
- EP-A- 0 346 006
- EP-A- 0 490 193
- EP-A- 1 129 709
- WO-A-93/20850
- WO-A-95/04528
- WO-A-2008/064192
- FR-A- 2 914 187

## Description

### FIELD OF THE INVENTION

The present invention refers to an ibuprofen lysinate-based pharmaceutical composition combined with cyclodextrins and microcrystalline cellulose combined with sodium carboxymethylcellulose as oral suspension and to the preparation procedure thereof.

### BACKGROUND OF THE INVENTION

Fever and pain are symptoms of several childhood illnesses, which occur as a result of an alteration in the body, an infection and other causes.

Nowadays, there exist different drugs in the pharmaceutical market for treating these symptoms, most of which are ibuprofen- and paracetamol-based drugs. Ibuprofen therapeutic void is well-known between the drug administration and the beginning of the antipyretic and analgesic effect, being an objectionable limitation in the treatment of fever and pain and in the time that goes by between the drug administration and the decrease in body temperature and pain relief. Analgesics, antipyretics and anti-inflammatory drugs are not water soluble like ibuprofen; they leave certain areas of the gastric mucosa exposed for a long time to the histolesive action of high concentrations of the active principle.

Ibuprofen lysinate is a salt obtained through a process of chemical synthesis, from ibuprofen and lysine amino acid. One of the most important physicochemical transformations underwent by ibuprofen when saltified with lysine is that it becomes a substance with high solubility in a watery solution since it is quickly and completely water-soluble. This means a more rapid and homogeneous gastrointestinal absorption which transforms the pharmacokinetic properties of ibuprofen and adds differential and advantageous therapeutic characteristics, although its organoleptic taste characteristics become worse.

One of the main advantages of ibuprofen lysinate is that, being an orally administered soluble salt, it homogeneously disperses over a wider gastric surface, enabling, on the one hand, a more rapid and homogeneous absorption, and on the other hand, a noticeably smaller incidence of side effects associated to analgesics, antipyretics and anti-inflammatory drugs which are not water soluble. These advantages translate into a better kinetic and tolerance profile for ibuprofen lysinate compared to ibuprofen, and consequently, into better clinical results since they allow a faster therapeutic action, which is a critical property for treating fever and pain, and a less harmful effect on gastric mucosa, which is a differential and advantageous difference compared to ibuprofen. The combination of ibuprofen lysinate and beta-cyclodextrins improves these properties and solves the problem of palatability producing an acceptable administration.

EP1129709 refers to an ibuprofen-based pharmaceutical composition in the form of powder, whose active component is ibuprofen lysinate combined with beta-cyclodextrins. This patent presents the advantages described of ibuprofen lysinate but excludes its administration to the pediatric population, since drug dosage for this population is carried out according to their body weight and, in oral administration the only pharmaceutical forms which enable to administer a variable dose are solutions or suspensions.

EP0346006 discloses a dispersion of beta cyclodextrin with ibuprofen salt, such as a lysine salt, associated with pharmaceutically diluents or carriers.

WO2008/064192 discloses a suspension comprising NSAID's derivative such as ibuprofen or its pharmaceutically acceptable salts, for example lysine salt of ibuprofen. The suspension can also comprise vehicles like preservatives, sweeteners, suspending agents or structuring agents like cyclodextrins.

In the market there exist different ibuprofen suspensions; however, none of them contains as active component ibuprofen lysinate, which is a different active principle from ibuprofen due to its significant differences as regards safety and efficacy, and they normally contain sugar, excluding their administration to patients suffering from diabetes.

There is then a need to find a way to orally administer ibuprofen lysinate to the pediatric population so that this population can benefit from the described therapeutic advantages of this active component compared to analgesics, antipyretics and anti-inflammatory drugs which are not water soluble.

### DESCRIPTION OF THE INVENTION

The present invention refers to an ibuprofen lysinate combined with cyclodextrins, and microcrystalline cellulose combined with sodium carboxymethylcellulose as colloidal agent, in the form of oral suspension, with analgesic, antipyretic and anti-inflammatory activity, not containing sucrose, having a variable dosage and being adaptable to the patient's weight so that it is intended to the pediatric population and suitable for patients suffering from diabetes or fructose/sucrose intolerance.

Thus, a first aspect of the present invention refers to a pharmaceutical composition containing ibuprofen lysinate combined with cyclodextrins, and microcrystalline cellulose combined with sodium carboxymethylcellulose as colloidal agents, and pharmaceutically acceptable excipients in the form of oral suspension.

In a more specific aspect, the pharmaceutical composition contains ibuprofen lysinate combined with cyclodextrins, and microcrystalline cellulose combined with sodium carboxymethylcellulose as colloidal agents, and pharmaceutically acceptable excipients in the form of oral suspension and it does not contain sucrose.

In a more specific embodiment, the cyclodextrins are beta-cyclodextrins, and in another specific embodiment, the cyclodextrins are hydroxypropyl beta-cyclodextrins.

In a more specific aspect, the ibuprofen lysinate is combined with beta-cyclodextrin with a weight ratio of ibuprofen lysinate/beta-cyclodextrin comprised between 1:1 and 1:5.

In the present invention by "ibuprofen lysinate combined with beta-cyclodextrin" we refer to ibuprofen lysinate encapsulated in the beta-cyclodextrins.

In another more specific aspect, the pharmaceutically acceptable excipients contained in the pharmaceutical composition of the invention, are selected from the group formed by colloidal agents, preservative agents, diluting agents, sweetening agents, aromatic agents and artificial colors.

In another more specific aspect, the colloidal agent is comprised between 0.4 and 3% in weight. In another more specific aspect, the colloidal agent is microcrystalline cellulose combined with sodium carboxymethylcellulose.

In another more specific aspect, the preservative agents of the pharmaceutical composition of the present invention are comprised between 0.02 and 2% in weight. In another more specific aspect, the preservative agents are selected among preservatives like paraben or potassium sorbate. In another more specific aspect, the preservative agents are combinations of methylparaben, ethylparaben, propylparaben and potassium sorbate.

In another more specific aspect, the sweetening agent of the pharmaceutical composition of the present invention is comprised between 0.10 and 0.20% in weight; in another more specific aspect, the sweetening agent is sodium saccharin, sodium cyclamate and aspartame.

In another more specific aspect, the pharmaceutical composition of the present invention contains fruit of the forest as aromatic agent.

In another more specific aspect, the pharmaceutical composition of the present invention contains allura red AC as artificial color.

A second aspect of the present invention refers to a procedure for preparing the pharmaceutical composition of the present invention which comprises the following stages:
a) encapsulation of ibuprofen lysinate in cyclodextrins. In a more specific aspect, the cyclodextrins are beta-cyclodextrins. In another more specific aspect, the encapsulation of ibuprofen lysinate is carried out through sifting and ultrarapid mixing of ibuprofen lysinate and beta-cyclodextrins in a 1:1-1:5 ratio during 1 to 20 minutes,
b) solubilization of preservatives in propylene glycol or alternatively, in purified water. In a more specific aspect, the preservatives are selected among preservatives like paraben and potassium sorbate. In a more specific aspect, the solubilization is carried out at a temperature between 60-100°C,
c) refrigeration of the mix of preservatives in purified water up to a temperature between 25-37°C,
d) addition into stage c) of the microcrystalline cellulose and sodium carboxymethylcellulose and mixing at high speed during 15 to 60 minutes to form the suspension,
e) addition into stage d) of the diluting agents, sweetening agents, ibuprofen lysinate-beta-cyclodextrin, aromatic agents, artificial colors and purified water qsp. In a more specific aspect, the diluting agents are maltitol and sorbitol, in another more specific aspect, the sweetening agent is sodium saccharin, sodium cyclamate or aspartame; in another more specific aspect, the aromatic agent is fruit of the forest; in another more specific aspect, the artificial color is Allura red,
f) filtration

### DETAILED DESCRIPTION OF THE INVENTION

Ibuprofen lysinate pharmaceutical composition combined with cyclodextrins (Table 1)

**Table 1**

| Pharmaceutical composition | Percentages in weights |
|---|---|
| Ibuprofen lysinate | 2-5 |
| Cyclodextrin | 2- 20 |
| Colloidal agent | 0.4-3 |
| Preservative agents | 0.02-2 |
| Diluting agents | 2-20 |
| Sweetening agents | 0.10-0.20 |
| Aromatic agents | 0.10-0.50 |
| Artificial colors | 0.006-0.009 |
| Purified water q.s.p. | 100 ml |

### Example 1: Ibuprofen lysinate pharmaceutical composition combined with beta-cyclodextrins (Table 2)

**Table 2**

| Pharmaceutical composition | Percentages in weights |
|---|---|
| Ibuprofen lysinate | 2-5 |
| Beta-cyclodextrin | 2- 20 |
| Microcrystalline cellulose/sodium carboxymethylcellulose | 0.4-3 |
| Methylparaben, propylparaben, ethylparaben | 0.1 - 0,5 |
| Maltitol | 5.00-20.00 |
| Sorbitol | 2.00-3.50 |
| Sodium saccharin | 0.10-0.20 |
| Fruit of the forest aromatic agent | 0.10-0.50 |
| Allura red AC | 0.006-0.009 |
| Purified water q.s.p. | 100 ml |

### Example 2: Ibuprofen lysinate pharmaceutical composition combined with cyclodextrins (Table 3)

**Table 3**

| Pharmaceutical composition | Percentages in weights |
|---|---|
| Ibuprofen lysinate | 2 |
| Beta-cyclodextrin | 6.5 |
| Microcrystalline cellulose/sodium carboxymethylcellulose | 1 |
| Methylparaben, propylparaben, ethylparaben | 0.2 |
| Maltitol | 12 |
| Sorbitol | 2 |
| Sodium saccharin | 0.12 |
| Fruit of the forest aromatic agent | 0.15 |
| Allura red AC | 0.006 |
| Purified water q.s.p. | 100 ml |

### Example 3: Ibuprofen lysinate pharmaceutical composition combined with cyclodextrins (Table 4)

**Table 4**

| Pharmaceutical composition | Percentages in weights |
|---|---|
| Ibuprofen lysinate | 4 |
| Beta-cyclodextrin | 16 |
| Microcrystalline cellulose/sodium carboxymethylcellulose | 0.6 |
| Propylparaben | 0.04 |
| Potassium sorbate | 1.5 |
| Propylene glycol | 2 |
| Maltitol | 9 |
| Sorbitol | 3.5 |
| Aspartame | 0.15 |
| Fruit of the forest aromatic agent | 0.2 |
| Allura red AC | 0.007 |
| Purified water q.s.p. | 100 ml |

### Example 4: Ibuprofen lysinate pharmaceutical composition combined with beta-cyclodextrins (Table 5)

**Table 5**

| Pharmaceutical composition | Percentages in weights |
|---|---|
| Ibuprofen lysinate | 3.5 |
| Beta-cyclodextrin | 10 |
| Microcrystalline cellulose/sodium carboxymethylcellulose | 0.8 |
| Methylparaben, propylparaben, ethylparaben | 0.25 |
| Potassium sorbate | 1 |
| Propylene glycol | 3 |
| Maltitol | 9 |
| Sorbitol | 2 |
| Sodium saccharin | 0.20 |
| Fruit of the forest aromatic agent | 0.14 |
| Allura red AC | 0.007 |
| Purified water q.s.p | 100 ml |

### Example 5: Method for preparing ibuprofen lysinate pharmaceutical composition.

The procedure for preparing the ibuprofen lysinate pharmaceutical composition of the present invention was carried out according to the following steps:
The ibuprofen lysinate is encapsulated through sifting and ultrarapid mixing of ibuprofen lysinate and cyclodextrins in a 1:1-1:5 ratio during 1 to 20 minutes. Next, there is a solubilization of preservatives in purified water at a temperature between 60-100°C; said solubilization can be made in propylene glycol. Said mix (preservatives in purified water) was refrigerated until up to a temperature between 25-27°C. Later, the colloidal agent is added to said mix and mixed at high speed during 15 to 60 minutes, until the suspension was formed. The diluting agents, the sweetening agent, the ibuprofen lysinate-cyclodextrin, the aromatic agent, the artificial color and purified water qsp were added to said mix and all components were mixed. Finally, the mix was filtered.

### Example 6: Procedure for preparing the ibuprofen lysinate pharmaceutical composition described in example 2.

a) encapsulation of ibuprofen lysinate through sifting and ultrarapid mixing of ibuprofen lysinate and beta-cyclodextrins in a 1:1-1:5 ratio during 1 to 20 minutes.
b) solubilization of methylparaben, ethylparaben and propylparaben in purified water at a temperature between 60-100°C.
c) refrigeration of the mix of preservatives in purified water up to a temperature between 25-37°C.
d) addition into stage c) of the microcrystalline cellulose and sodium carboxymethylcellulose and mixing at high speed during 15 to 60 minutes to form the suspension.
e) addition into stage d) of the following compounds:
   - maltitol
   - sorbitol
   - sodium saccharin
   - fruit of the forest aromatic agent
   - allura red AC
   - purified water qsp
f) filtration

### Example 7: Procedure for preparing the ibuprofen lysinate pharmaceutical composition described in example 3.

a) encapsulation of ibuprofen lysinate through sifting and ultrarapid mixing of ibuprofen lysinate and cyclodextrins in a 1:1-1:5 ratio during 1 to 20 minutes.
b) solubilization of propylparaben and potassium sorbate in propylene glycol.
c) addition into stage b) of the microcrystalline cellulose and sodium carboxymethylcellulose and mixing at high speed during 15 to 60 minutes to form the suspension.
d) addition into stage c) of the following compounds:
   - maltitol
   - sorbitol
   - aspartame
   - fruit of the forest aromatic agent
   - allura red AC
   - purified water qsp
f) filtration

### Example 8: Procedure for preparing the ibuprofen lysinate pharmaceutical composition described in example 4.

a) encapsulation of ibuprofen lysinate through sifting and ultrarapid mixing of ibuprofen lysinate and cyclodextrins in a 1:1-1:5 ratio during 1 to 20 minutes.
b) solubilization of methylparaben, propylparaben, ethylparaben and potassium sorbate in propylene glycol.
c) addition into stage b) of the microcrystalline cellulose and sodium carboxymethylcellulose and mixing at high speed during 15 to 60 minutes to form the suspension.
d) addition into stage c) of the following compounds:
   - maltitol
   - sorbitol
   - sodium saccharin
   - fruit of the forest aroma
   - allura red AC
   - purified water qsp
f) filtration

## Claims

1. Pharmaceutical composition containing ibuprofen lysinate combined with cyclodextrins, microcrystalline cellulose combined with sodium carboxymethylcellulose as colloidal agent to form the suspension and pharmaceutically acceptable excipients, in the form of oral suspension.

2. Pharmaceutical composition according to claim 1, **characterized in that** the cyclodextrins are beta-cyclodextrins or hydroxypropyl beta-cyclodextrins.

3. Pharmaceutical composition according to any of claims 1 or 2, **characterized in that** the weight ratio between ibuprofen lysinate and cyclodextrin is comprised between 1:1 and 1:5.

4. Pharmaceutical composition according to any of the preceding claims, **characterized in that** the pharmaceutically acceptable excipients are selected from the group formed by preservative agents, maltitol and sorbitol as diluting agents, sweetening agents, aromatic agents, and artificial colors.

5. Pharmaceutical composition according to any of claims 1 to 4, **characterized in that** the colloidal agent has a weight comprised between 0.4 and 3%.

6. Pharmaceutical composition according to claim 4, **characterized in that** the preservative agent is comprised between 0.02 and 2% in weight.

7. Pharmaceutical composition according to claim 4, **characterized in that** the preservative agent is selected among preservatives like paraben or potassium sorbate.

8. Pharmaceutical composition according to claim 4, **characterized in that** the preservative agents are combinations of potassium sorbate, methylparaben, ethylparaben and propylparaben.

9. Pharmaceutical composition according to claim 4, **characterized in that** the diluting agents are comprised between 2 and 20% in weight.

10. Procedure for preparing a pharmaceutical composition according to any of claims 1 to 9, comprising the following stages:
a) encapsulation of ibuprofen lysinate in the cyclodextrins;
b) solubilization of a preservative in purified water or in propylene glycol;
c) addition into stage b) of the microcrystalline cellulose combined with sodium carboxymethylcellulose and mixing at high speed to form the suspension;
d) addition into stage c) of diluting agents, sweetening agents, the ibuprofen lysinate-cyclodextrin, aromatic agents, artificial colors and purified water qsp.
e) filtration.

11. Procedure according to claim 10, **characterized in that** the stage a) of ibuprofen lysinate encapsulation in the cyclodextrins is carried out through sifting and ultrarapid mixing of ibuprofen lysinate and beta-cyclodextrins in a 1:1-1:5 ratio.

## Patentansprüche

1. Pharmazeutische Zusammensetzung enthaltend Ibuprofenlysinat in Kombination mit Cyclodextrinen, mikrokristalline Cellulose in Kombination mit Natriumcarboxymethylcellulose als kolloidales Mittel um die Suspension zu bilden und pharmazeutisch akzeptable Hilfsstoffe, in Form von einer oralen Suspension.

2. Pharmazeutische Zusammensetzung nach Anspruch 1, **dadurch gekennzeichnet, dass** die Cyclodextrine Beta-Cyclodextrine oder Hydroxypropyl-Beta-Cyclodextrine sind.

3. Pharmazeutische Zusammensetzung nach einem der Ansprüche 1 oder 2, **dadurch gekennzeichnet, dass** das Gewichtsverhältnis zwischen Ibuprofenlysinat und Cyclodextrin zwischen 1:1 und 1:5 liegt.

4. Pharmazeutische Zusammensetzung nach einem der vorhergehenden Ansprüche, **dadurch gekennzeichnet, dass** die pharmazeutisch akzeptablen Hilfsstoffe aus der Gruppe ausgewählt werden, gebildet aus Konservierungsmitteln, Maltit und Sorbit als Verdünnungsmittel, Süßstoffen, Duftmitteln, und künstlichen Farben.

5. Pharmazeutische Zusammensetzung nach einem der Ansprüche 1 bis 4, **dadurch gekennzeichnet, dass** das kolloidale Mittel ein Gewicht, das zwischen 0,4 und 3% liegt, aufweist.

6. Pharmazeutische Zusammensetzung nach Anspruch 4, **dadurch gekennzeichnet, dass** das Konservierungsmittel zwischen 0,02 Gew.-% und 2 Gew.-% liegt.

7. Pharmazeutische Zusammensetzung nach Anspruch 4, **dadurch gekennzeichnet, dass** das Konservierungsmittel unter Konservierungsmitteln wie Paraben oder Kaliumsorbat ausgewählt wird.

8. Pharmazeutische Zusammensetzung nach Anspruch 4, **dadurch gekennzeichnet, dass** die Konservierungsmittel Kombinationen aus Kaliumsorbat, Methylparaben, Ethylparaben und Propylparaben sind.

9. Pharmazeutische Zusammensetzung nach Anspruch 4, **dadurch gekennzeichnet, dass** die Verdünnungsmittel zwischen 2 Gew.-% und 20 Gew.-% liegen.

10. Verfahren zur Herstellung einer pharmazeutischen Zusammensetzung nach einem der Ansprüche 1 bis 9, umfassend die folgenden Schritte:
a) das Verkapseln von Ibuprofenlysinat in den Cyclodextrinen;
b) das Solubilisieren von einem Konservierungsmittel in gereinigtem Wasser oder in Propylenglycol;
c) das Hinzufügen zum Schritt b) der mikrokristallinen Cellulose in Kombination mit Natriumcarboxymethylcellulose und das Mischen bei hoher Geschwindigkeit um die Suspension zu bilden;
d) das Hinzufügen zum Schritt c) der Verdünnungsmittel, Süßstoffe, des Ibuprofenlysinat-Cyclodextrins, der Duftmittel, der künstlichen Farben und des gereinigten Wassers s.q.
e) das Filtrieren.

11. Verfahren nach Anspruch 10, **dadurch gekennzeichnet, dass** der Schritt a) des Verkapselns von Ibuprofenlysinat in den Cyclodextrinen durch Sieben und ultraschnelles Mischen von Ibuprofenlysinat und Beta-Cyclodextrinen in einem 1:1-1:5 Verhältnis durchgeführt wird.

## Revendications

1. Composition pharmaceutique contenant de lysinate d'ibuprofène combiné avec des cyclodextrines, de la cellulose microcristalline combinée avec du carboxyméthylcellulose de sodium comme agent colloïdal pour former la suspension et les excipients pharmaceutiquement acceptables, sous forme de suspension orale.

2. Composition pharmaceutique selon la revendication 1, **caractérisée en ce que** les cyclodextrines sont des béta-cyclodextrines ou des hydroxypropyl-béta-cyclodextrines.

3. Composition pharmaceutique selon l'une quelconque des revendications 1 ou 2, **caractérisée en ce que** le rapport de pois entre la lysinate d'ibuprofène et la cyclodextrine est compris entre 1 : 1 et 1 : 5.

4. Composition pharmaceutique selon l'une quelconque des revendications précédentes, **caractérisée en ce que** les excipients pharmaceutiquement acceptables sont choisis parmi le groupe formé d'agents conservateurs, de maltitol, et de sorbitol comme agents de dilution, agents édulcorants, et agents artificiels.

5. Composition pharmaceutique selon l'une quelconque des revendications 1 ou 4, **caractérisée en ce que** l'agent colloïdal a un poids compris entre 0,4 et 3%.

6. Composition pharmaceutique selon la revendication 4, **caractérisée en ce que** l'agent conservateur est compris entre 0,02 et 2% en poids.

7. Composition pharmaceutique selon la revendication 4, **caractérisée en ce que** l'agent conservateur est choisi parmi les conservateurs comme le parabène et le sorbate de potassium.

8. Composition pharmaceutique selon la revendication 4, **caractérisée en ce que** les agents conservateurs sont des combinaisons de sorbate de potassium, de méthylparabène, d'éthylparabène et de propylparabène.

9. Composition pharmaceutique selon la revendication 4, **caractérisée en ce que** les agents de dilution sont compris entre 2 et 20% en poids.

10. Procédé de préparation d'une composition pharmaceutique selon l'une quelconque des revendications 1 à 9, comprenant les étapes suivantes :
a) encapsulation du lysinate d'ibuprofène dans les cyclodextrines ;
b) solubilisation d'un conservateur dans de l'eau purifiée ou dans du propylène glycol ;
c) addition lors de l'étape d) de la cellulose microcristalline combinée avec de la carboxyméthylcellulose de sodium et mélange à grande vitesse pour former la suspension.
d) addition lors de l'étape c) d'agents de dilution, d'agents édulcorants, le lysinate d'ibuprofène-cyclodextrine, d'agents aromatiques, de colorants artificiels et d'eau purifiée qsp.
e) filtration.

11. Procédé selon la revendication 10, caractérisé en que ce l'étape a) d'encapsulation de lysinate d'ibuprobène dans les cyclodextrines est réalisée par tamisage et mélange ultra-rapide du lysinate d'ibuprofène et des béta-cyclodextrines dans un rapport de 1 : 1 - 1 : 5.
